**Europäisches Patentamt**

**European Patent Office**

**Office européen des brevets**

(19)

(11) Veröffentlichungsnummer: **0 166 233**
A2

(12) # EUROPÄISCHE PATENTANMELDUNG

(21) Anmeldenummer: **85106446.9**

(51) Int. Cl.⁴: **G 01 N 33/531**

(22) Anmeldetag: **24.05.85**

(30) Priorität: **26.05.84 DE 3419782**

(43) Veröffentlichungstag der Anmeldung: **02.01.86**
**Patentblatt 86/1**

(84) Benannte Vertragsstaaten: **AT BE CH DE FR GB IT LI LU NL SE**

(71) Anmelder: **GÖDECKE AKTIENGESELLSCHAFT,**
**Salzufer 16, D-1000 Berlin 10 (DE)**

(72) Erfinder: **Keck, Rolf, Dr., August-Kirsch-Strasse 13,**
**D-6922 Meckesheim (DE)**
Erfinder: **Ding, Otto, Kreisental 7, D-6921 Epfenbach**
**(DE)**

(54) **Sphärische Proteinpartikel mit immunadsorbierenden Eigenschaften und Verfahren zu deren Herstellung.**

(57) Es wird ein Verfahren zur Herstellung von sphärischen Proteinpartikeln durch gezielte Polymerisation von Proteinen mittels Chlorameisensäurealkylestern in wäßriger Lösung beschrieben. Die erhaltenen sphärischen Proteinpartikel besitzen eine Größe von 1–50 µm und eignen sich z.B. als Träger für diagnostische Testsysteme.

## B E S C H R E I B U N G

Aus The Journal of Biological Chemistry 242, No. 7, S. 1651 (1967) sind wasserunlösliche biologisch aktive Proteinpolymere bekannt, die immunadsorbierende Eigenschaften besitzen. Mit solchen Proteinpolymeren ist es bereits gelungen, spezifische Antikörper aus Gemischen einer Reihe von Proteinen zu isolieren. Es ist auch gelungen, aus Proteinlösungen unerwünschte Antigene und Antikörper zu enfernen. Nach dem beschriebenen Verfahren zur Herstellung dieser Proteinpolymere mittels Chlorameisensäureestern erhält man gelförmige Produkte, die anschließend mechanisch zerteilt und homogenisiert werden müssen, wenn man immunadsorbierende Proteinpartikel erhalten will.

Die mechanische Zerteilung der erhaltenen Proteine führt jedoch, wie Versuche ergaben, zu unregelmäßig geformten Partikeln, die aufgrund der unebenen Oberfläche dazu neigen, sich ineinander zu "verhaken", was besonders bei der Lagerung zu unerwünschter Aggregation und schließlich zur Klumpenbildung führt. So hergestellte Proteinpartikel sind daher nicht lagerfähig und müßten vor jeder Verwendung erneut homogenisiert werden. Dies ist aber, abgesehen vom Zeitaufwand, auch deshalb ungünstig, weil jede mechanische Homogenisierung insbesondere durch Ultraschall zu Aktivitätsverlusten führt.

Aufgabe der vorliegenden Erfindung ist es, die Polymerisation von Proteinen so zu lenken, daß unmittelbar annähernd sphärische Teilchen mit immunadsorbierenden Eigenschaften von in etwa vorherbestimmbarem, gleichmäßigem Durchmesser entstehen. Eine weitere Aufgabe der Erfindung ist es, durch Polymerisation von Proteinen, Suspensionen von Teilchen mit immunadsorbierenden Eigenschaften herzustellen, die sich aufgrund der Form und Größe der suspendierten Teilchen unmittelbar für diagnostische Zwecke oder zur präparativen Adsorbtion von bestimmten Proteinen verwenden lassen und damit z.B. die bisher verwendeten beladenen Latex-Partikel weitgehend zu ersetzen vermögen.

Es wurde gefunden, daß man bei der Polymerisation von Proteinen mittels Chlorameisensäureestern überraschend reproduzierbar wasserunlösliche sphärische Teilchen mit gleichmäßigem Durchmesser von etwa 1 μm bis etwa 10 μm in wässriger Suspension erhält, wenn man die Polymerisation von Proteinen unter folgenden Bedingungen durchführt:

1.) Der Proteingehalt der zu polymerisierenden Proteinlösung muß im Bereich von 0,5 bis 4,5 Gew.% liegen, bevorzugt im Bereich von 2 bis 3 Gew. % liegen. Der optimale Wert liegt bei 2,5 Gew. %.

2.) Der pH-Wert der Lösung darf während der Polymerisation den Bereich von 5,0 bis 6,0 nicht verlassen. Der günstigste pH-Wert liegt bei 5,5.

3.) Die Polymerisation muß im Temperaturbereich von 20-40$^{\circ}$C, bevorzugt bei 28-32$^{\circ}$C durchgeführt werden.

4.) Während der Polymerisation muß die Lösung sehr stark gerührt werden (Magnetrührer bei hoher Tourenzahl).

5.) Das Reaktionsgefäß sollte mit einer geerdeten Elektrode ausgestattet sein, die in die Proteinlösung eintaucht, um statische Aufladungen während des Polymerisationsprozesses abzuleiten.

Gemäß dem bekannten Stand der Technik erhält man selbst im erfindungsgemäß günstigsten pH-Bereich von 5,3 bis 5,6 bei der Polymerisation von Human-Serum-albumin oder Transferrin lediglich gelförmige Produkte (vgl. Tabelle 1 auf S. 1654). Den Beispielen auf S. 1652, Spalte 2 ist zu entnehmen, daß bei der Reaktion nur sanft (gentle) gerührt werden soll.

Umfangreiche und über einen Zeitraum von über 10 Jahren durchgeführte Versuchsreihen haben gezeigt, daß die Parameter 1 bis 4 kritisch sind und eingehalten werden müssen. Anderenfalls werden, wie beim Verfahren gemäß J. Biol. Chem., lediglich gelartige Produkte erhalten oder es wird die Hydrolyse des Chlorameisensäureesters so stark begünstigt, daß er nicht mehr in der Lage ist mit den Proteinen der Lösung zu reagieren.

Auch die Bedingung 5 ist einzuhalten, wenn reproduzierbare Ergebnisse erzielt werden sollen. Das Maß der elektrostatischen Aufladung der Teilchen ist nämlich von Faktoren abhängig, die nicht ohne weiteres ermittelt werden können. Gemäß den durchgeführten Versuchsreihen liegt sogar eine deutlich erkennbare Wetterabhängigkeit vor, so daß zwar die Polymerisationsreaktion an gewissen Tagen auch ohne Erdung befriedigend ablaufen kann, während an anderen Tagen mit denselben Reagenzien und derselben apparativen Ausstattung die gewünschten sphärischen Teilchen erst erhalten werden können, wenn in die Lösung eine geerdete Elektrode eingebracht wird. Die elektrostatischen Effekte führen offensichtlich zu einer starken Abstoßung zwischen den polymerisierenden Proteinmolekülen und daher zu keinem geeigneten Polymerisat.

Der Proteingehalt beträgt bei den bekannten Reaktionen 5-10 % (J. Biol.-Chem. Beispiele I-III). Es wurde gefunden, daß diese Konzentration zu hoch ist und daß diese hohe Konzentration eine der Ursachen für die unerwünschte Gelbildung ist. Es wurde weiterhin gefunden, daß der Konzentrationsbereich sehr kritisch ist und mit nur 2 % Abweichung nach oben und unten bei 2,5 Gew. % liegen muß. Wählt man die Konzentration höher, so bilden sich gelartige Produkte, wählt man sie niedriger, so wird die Reaktion durch die dann überwiegende Hydrolyse des Chlorameisensäureesters unterbunden. Ein bevorzugter Bereich liegt bei 2,5 ± 0,5 %. Die günstigste Konzentration liegt bei 2,5 Gew. %.

Ähnlich kritisch ist der pH-Bereich. Bei zu hohem pH (> 6) überwiegt die Hydrolysereaktion des als Polymerisationsreagenz eingesetzten Chlorameisensäureesters, so daß die Reaktion "einschläft". Bei zu niedrigem pH-Wert (< 5) entstehen unregelmäßige Partikel und in ungünstigen Fällen, d.h. wenn auch die anderen Parameter außerhalb des günstigsten Bereichs liegen, gelartige Produkte.

Auch der Temperaturbereich von 28 bis 32°C sollte möglichst genau mittels eines rasch regelnden Thermostaten eingehalten werden. Jenseits der Grenztemperaturen von 20°C bzw. 40°C wird die Reaktionskinetik jeweils so ungünstig beeinflußt, daß die gewünschten sphärischen Proteinpartikel nicht mehr entstehen.

Es ist auch notwendig, so stark wie möglich zu rühren, um jederzeit eine absolut gleichmäßige Verteilung des Chlorameisensäureesters in der Proteinlösung zu erzielen. Es sollte so stark gerührt werden, wie die Schaumbildung dies zuläßt. Gepufferte Proteinlösungen, wie z.B. Seren, haben die unangenehme Eigenschaft leicht zu schäumen, so daß auch durch die Wahl des Reaktionsgefäßes und des Rührers die Schaumbildung möglichst unterdrückt werden sollte. Hierbei hat es sich als günstig erwiesen, einen Magnetrührer zu verwenden, dessen Länge möglichst dem Innendurchmesser des runden Reaktionsgefäßes entspricht, so daß zwischen der Innenwand und den Enden des Rührstabes nur wenig Raum bleibt. Die Rührgeschwindigkeit kann dann beispielsweise 400-1000 UpM betragen. Die Erdung des Reaktionsgefäßes kann durch eine in die Wandung eingeschmolzene Platinelektrode (Platindraht) bewirkt werden. Es ist jedoch auch möglich, den Boden des Reaktionsgefäßes und ggf. den Rührer aus Edelmetall oder Edelstahl auszubilden und die zur Beobachtung des Reaktionsverlaufs vorzugsweise durchsichtige Wand wasserdicht mit dem metallenen Boden zu verbinden und den metallenen Boden zu erden.

Erfahrungsgemäß genügt aber bereits eine relativ geringe Metalloberfläche z.B. in Form eines kurzen geerdeten Platindrahts, um die unerwünschten elektrostatischen Störungen auszuschalten und damit die Reproduzierbarkeit des Verfahrens sicherzustellen.

Gegenstand der vorliegenden Erfindung ist demnach ein Verfahren zur Herstellung von sphärischen Proteinpartikeln mit immunadsorbierenden Eigenschaften durch Polymerisation von Proteinen mittels eines Esters der Chlorameisensäure, dadurch gekennzeichnet, daß man die Reaktion mit einer gepufferten Proteinlösung mit einem Proteingehalt von 0,5 bis 4,5 Gew. % bei einem pH-Wert zwischen 5,0 und 6,0 und bei einer Temperatur zwischen 20°C und 40°C unter starkem Rühren durchführt.

Ein weiterer Gegenstand sind sphärische Proteinpartikel mit einem Durchmesser von 1 μm bis 50 μm, die nach dem oben beschriebenen Verfahren hergestellt sind. Bevorzugt ist ein Bereich von 1 μm bis 10 μm.

Im Einzelnen wird das Verfahren wie folgt durchgeführt:

Eine proteinhaltige Flüssigkeit, wie z.B. menschliches oder tierisches Serum oder Milch wird auf einen Proteingehalt von 0,5 bis 4,5 Gew. % verdünnt und mit einem Puffer auf einen pH-Wert von 5,0 bis 6,0 eingestellt. Die verdünnte und gepufferte Lösung wird dann in einem Standzylinder thermostatisiert (20-40°C, vorzugsweise 30 ± 2°C) und mittels eines Magnetrührers so stark gerührt, daß die Schaumbildung in erträglichen Grenzen gehalten wird. Das Reaktionsgefäß selbst ist, wie oben beschrieben, geerdet. Mittels einer Dosiervorrichtung werden nun in einer Gesamtzeit von etwa 5 Minuten 2 bis 6, vorzugsweise etwa 3 μMol Chlorameisensäurealkylester pro mg Protein zugegeben. Die Zugabe kann entweder ununterbrochen oder in Intervallen erfolgen. Es hat sich als günstig erwiesen, die Reaktion zunächst mit 1,5 μMol/mg Protein Chlorameisensäurealkylester zu starten und die zweite Hälfte des Reagenz erst 15-30 Minuten nach der ersten Zugabe zuzugeben. Zu Beginn des Versuchs wird also 2,5 Minuten zugegeben, nach 15-30 Minuten erfolgt eine weitere Zugabe von 2,5 Minuten, so daß die Gesamtzugabezeit 5 Minuten beträgt.

- 6 -                                    0166233

Als Chlorameisensäurealkylester kommen alle für die Peptidsynthese brauchbaren niederen Alkylester, wie z.B. der Methyl- oder Ethylester infrage.

Während der gesamten Reaktionszeit wird der pH-Wert der Lösung ununterbrochen gemessen und durch Titration mit 0,5 n Natronlauge auf dem Sollwert gehalten. Wenn der pH-Wert ohne weitere Zugabe von Natronlauge konstant bleibt, wird die Größe der gebildeten Proteinpartikel durch mikroskopische Untersuchung kontrolliert und die Reaktion bei Erreichen der Sollgröße durch Zugabe von 3,3 %iger Ammoniaklösung gestoppt. Hierbei steigt der pH-Wert der Lösung auf 9,0. Es wird noch 30 Minuten weitergerührt, dann wird 20 Minuten bei 3000-5000 g zentrifugiert und das Zentrifugat jeweils nach dem Abdekantieren dreimal mit Triäthanolaminhydrochlorid Puffer (pH 7,2, 0,005 m) gewaschen. Anschließend wird mit Wasser gewaschen, dem zur Stabilisation 0,1 % Natriumazid und 0,1 % Benzoesäure zugegeben werden. Schließlich werden die erhaltenen sphärischen Partikel in demselben Medium resuspendiert und gelagert.

Die Menge X der jeweils einzusetzenden Protein-Ausgangslösung wird wie folgt errechnet:

$$X = \frac{G \times V}{P}$$

G = Proteingehalt in % des Endvolumens (ml)
V = Endvolumen (ml)
P = Proteingehalt der Ausgangslösung (%)

Ermittelt man z.B. analytisch einen Proteingehalt von 7,5 % (P) und strebt ein Endvolumen (V) von 30 ml an so ergibt sich für einen Proteingehalt von 2,5 %:

$$X = \frac{2,5 \times 30}{7,5} = \underline{10}$$

Es sind in diesem Fall also 10 ml Proteinlösung mit Wasser und Pufferlösung auf 30 ml zu verdünnen.

Bei der Verdünnung geht man praktisch so vor, daß man das Ausgangsprotein mit Wasser auf ein Volumen von $\frac{V}{2}$ verdünnt und zu dieser Verdünnung $\frac{V}{2}$ 0,15 molaren $NaH_2 PO_4$-Puffer vom pH 5,5 zugibt. Es kann auch erst die Pufferlösung zugegeben und dann verdünnt werden. Da die pH-Einstellung kritisch ist, ist es notwendig das benutzte pH-Meter vor jedem Versuch zu eichen. Es sollte mit einer proteinverträglichen Elektrode mit einer Steilheit von mindestens 94 % gemessen werden.

Die folgenden Beispiele dienen der näheren Erläuterung der Erfindung:

B E I S P I E L   1

Anti-Human-Chorion-Gonadotropin-Partikel

In einem thermostatisierten (30°C) und geerdeten 100 ml-Standzylinder werden nacheinander folgende Komponenten gegeben und mittels Magnetrührer bei 700 UpM vermischt:

1.) 9,9 ml Anti-HCG-Serum mit einem durch Biuret-Reaktion ermittelten Proteingehalt von 7,6 %

2.) 15 ml $NaH_2PO_4$-Puffer (0,15 molar, pH 5,5)

3.) 5,1 ml Wasser

Der Ausgangs-pH-Wert bei 30° liegt bei 6,01.

Die Lösung wird zunächst 15 Minuten gerührt. Dann werden 120 $\mu l$ Chlorameisensäuremethylester zugegeben. Hierbei wird der pH-Wert des Reaktionsgemischs durch Zugabe von 0,5 n NaOH konstant auf 5,0 gehalten. Nach weiteren 5 Minuten werden nochmal 120 $\mu l$ Chlorameisensäuremethylester (CAM) zugegeben. Nach etwa 50 Minuten ist der NaOH-Verbrauch bei 2,6 ml beendet, die Reaktion wird durch Einstellen von pH 9,5 mit 3,3 % $NH_3$ und 30minütigem Rühren unter diesen Bedingungen gestoppt. Es wird dreimal durch Zentrifugation und Resuspension in Triethanolamin/HCl (0,005 m, pH 7,2 NaCl, Nichtionisches Detergens) gewaschen, anschließend noch einmal in 0,1 % Rinder-Serum-Albumin (=BSA), 0,1% $NaN_3$ gewaschen und im selben Medium (ca. 50 ml) aufgenommen.

Es wird mit HCG-Standard in einem direkten Slide-Agglutinationstest verglichen. Dies ergibt gute Ableseergebnisse zwischen 5 und über 1000 IU/ml (IU = Internationale Einheiten). Das Maximum der Agglutinationsreaktion liegt bei 100 IU/ml und kann wesentlich größere Agglutinate als bei Latextests ergeben. Das Reaktionsbild ist vergleichbar mit serologischen Agglutinationsbildern.

BEISPIEL 2

Anti-CRP-Partikel

In der beschriebenen Versuchsanordnung werden bei 850 UpM folgende Komponenten gemischt:

15 ml Na-Phosphatpuffer (pH 5,5  0,15 m)
13,15 ml Anti-CRP-Serum (CRP = C-reaktives-Protein)
1,85 ml $H_2O$

Ausgangs-pH-Wert bei $30^{\circ}C$: 6,02
Der Start der Polymerisation erfolgt durch Zugabe von 25 $\mu$l Chlorameisensäuremethylester (CAM), dann werden nach pH-Abfall insgesamt weitere 7 mal je 25 $\mu$l CAM unter automatischer pH-Stabilisierung auf pH 5,0 (Zugabe im Abstand von ca. 20 Minuten) zugegeben. Nachdem der NaOH-Verbrauch bei 1,64 ml beendet ist, wird die Polymerisation durch Einstellen von pH 9,0 und 30minütiges Rühren unter diesen Bedingungen gestoppt (3,3 % $NH_3$ als Base). Die Waschschritte und Resuspensionsbedingungen entsprechen denen des Beispiels 1.

Ein Test gegen eine CRP-Standardreihe zeigt positive Reaktionen von ca. 100 mg CRP/l bis unter 20 $\mu$g/l ($\hat{=}$ 20 ng/ml), also eine sehr breite Äquivalenzzone.

Sehr kleine, auf diese Weise hergestellte Partikel wurden in der beschriebenen Weise als Ausgangsmaterial für eine Weiterpolymerisation zu größeren Partikeln eingesetzt und zeigten dann gegen CRP-Standard positive Reaktionen im Bereich von ca. 100 mg/l bis ca. 2 mg CRP/l. Durch den Polymerisationsgrad ließ sich also die Lage der Äquivalenzzone beeinflussen.

## B E I S P I E L    3

### Human-IgG-Präparat

In der in Beispiel 1 beschriebenen Versuchsanordnung werden bei 900 UpM folgende Komponenten gemischt:

2,34 ml Human-IgG (16 % Eiweiß)

12,65 ml $H_2O$

15 ml Na-Phosphatpuffer 0,15 m pH 5,5

Ausgangs-pH-Wert bei $30^{\circ}$C: 6,15

Der Start der Polymerisation erfolgt durch Zugabe von 50 µl Chlorameisensäure-ethylester (CAE), danach werden unter Stabilisierung des pH-Wertes auf 5,0 noch 4 mal jeweils 50 µl, zum Schluß der Polymerisation noch einmal 25 µl CAE zugegeben. Nach 2,07 ml ist der NaOH-Verbrauch beendet, die Polymerisation wird durch Einstellen von pH 9,0 und 30minütiges Weiterrühren beendet. Die Wasch- und Resuspensionschritte entsprechen denen des Beispiels 1. Die Partikel zeigen positive Reaktionen mit rheumafaktorhaltigen Seren.

## B E I S P I E L    4

### Anti-Norfenefrin-Partikel

In der in Beispiel 1 beschriebenen Versuchsanordnung werden bei 700 UpM folgende Komponenten gemischt:

10,7 ml Anti-Norfenefrin-Globulin

4,3 ml $H_2O$

15 ml Na-Phosphatpuffer 0,15 m   pH 5,5

Ausgangs-pH-Wert bei 30°C: 5,7

Der Start der Polymerisation erfolgt durch Zugabe von 100 µl Chlorameisensäureethylester (CAE), danach werden nach pH-Stabilisierung auf pH 5,0 noch einmal 100 µl CAE zugegeben. Es werden insgesamt 2,84 ml NaOH verbraucht, die Reaktion wie in Beispiel 1 beschrieben beendet und die Partikel ebenso gewaschen und resuspendiert. Die Partikel zeigen gegen Norfenefrin-Albumin eine positive Reaktion bis in den Bereich um 10 µg/ml und sind (unter dem Mikroskop vermessen) durchschnittlich ca. 0,8 µ groß.

### B E I S P I E L   5

#### Milchpartikel

15 ml Milch
15 ml Na-Phosphatpuffer (0,15 m   pH 5,5)

werden mit 0,438 ml CAE unter automatischer pH-Stabilisation (pH 5,0) unter Rühren mit ca. 500 UpM zur Polymerisation gebracht. Nach Verbrauch von 8,23 ml NaOH ist die Reaktion beendet und die Polymerisation wird wie in Beispiel 1 beschrieben gestoppt. Die Partikelgröße liegt unter 10 µ.

### B E I S P I E L   6

#### Frischserumpartikel (human)

In der in Beispiel 1 beschriebenen Versuchsanordnung werden bei 700 UpM folgende Komponenten gemischt:

5 ml Frischserum (human)
25 ml Na-Phosphatpuffer (0,15 m   pH 5,5)

Ausgangs-pH-Wert: 4,67 bei 30°C

Unter den Bedingungen gemäß Beispiel 1 werden 290 µl CAM zum Starten der Reaktion eingesetzt, die Reaktion ist nach 20 Minuten und einem Verbrauch von 1,48 ml NaOH beendet. Die Aufarbeitung erfolgt wie in Beispiel 1 beschrieben. Die Partikelgröße ist recht einheitlich und beträgt ca. 30 µ (mikroskopisch vermessen).

Die Partikelsuspension wurde erfolgreich zur Entfernung einiger unspezifischer Antikörper aus Anti-Leichtkette-K (von Schafen) eingesetzt.

## B E I S P I E L   7

### Anti-Rinderserumalbumin-Partikel

In der in Beispiel 1 beschriebenen Versuchsanordnung werden folgende Komponenten gemischt:

9,2 ml Anti-Rinderserumalbumin vom Schaf, dialysiert
gegen 0,5 m $NaH_2PO_4$, pH 5,5
20,8 ml Na Phosphatpuffer (0,5 m, pH 5,5)

Ausgangs-pH-Wert: 5,64 bei 30°C

Unter den Bedingungen gemäß Beispiel 1 werden 210 µl CAM zum Starten der Reaktion eingesetzt; die Reaktion ist nach 40 Minuten und einem Verbrauch von 3,18 ml NaOH (0,5 m) beendet. Die Aufarbeitung erfolgt wie in Beispiel 1 beschrieben. Die Partikelgröße beträgt ca. 15 - 30 µm.

BEISPIEL 8

Anti-humanalbumin-Partikel

In der in Beispiel 1 beschriebenen Versuchsanordnung werden folgende Komponenten gemischt:

8,2 ml Anti-humanalbumin vom Schaf dialysiert gegen
0,5 m $NaH_2PO_4$, pH 5,5
21,8 ml Na Phosphatpuffer (0,5 m, pH 5,5)

Ausgangs-pH-Wert: 5,72 bei 30°C

Unter den Bedingungen gemäß Beispiel 1 werden 145 µl CAM zum Starten der Reaktion eingesetzt; die Reaktion ist nach 60 Minuten und einem Verbrauch von 1,48 ml Na OH (0,5 m) beendet. Die Aufarbeitung erfolgt wie in Beispiel 1 beschrieben. Die Partikelgröße beträgt ca. 10 - 15 µm.

BEISPIEL 9

Anti-CRP-Partikel

In der in Beispiel 1 beschriebenen Versuchsanordnung werden folgende Komponenten gemischt:

9,6 ml Anti-CRP vom Schaf dialysiert gegen
0,5 $NaH_2PO_4$, pH 5,5
20,4 ml Na-Phosphatpuffer (0,5 m, pH 5,5)

Ausgangs-pH-Wert: 5,68 bei 30°C

Unter den Bedingungen gemäß Beispiel 1 werden 200 µl CAM zum Starten der Reaktion eingesetzt; die Reaktion ist nach 60 Minuten und einem Verbrauch von 3,36 ml Na OH (0,5 m) beendet. Die Aufarbeitung erfolgt wie in Beispiel 1 beschrieben. Die Partikelgröße beträgt ca. 10 - 30 µm.

## B E I S P I E L   10

### Anti-$\beta_1$E-Partikel

In der in Beispiel 1 beschriebenen Versuchsanordnung werden folgende Komponenten gemischt:

12,0 ml Anti-$\beta_1$E vom Schaf

18 ml Na Phosphatpuffer (0,5 m, pH 5,5)

Ausgangs-pH-Wert: 5,69 bei 30$^{\circ}$C

Unter den Bedingungen gemäß Beispiel 1 werden 200 µl CAM zum Starten der Reaktion eingesetzt; die Reaktion ist nach 55 Minuten und einem Verbrauch von 2,66 ml Na OH (0,5 m) beendet. Die Aufarbeitung erfolgt wie in Beispiel 1 beschrieben. Die Partikelgröße beträgt ca. 20 - 30 µm.

B E I S P I E L    11

Anti-$\beta_1$A-Partikel

In der in Beispiel 1 beschriebenen Versuchsanordnung werden folgende Komponenten gemischt:

10 ml Anti-$\beta_1$A vom Schaf
20 ml Na Phosphatpuffer (0,5 m, pH 5,5)

Ausgangs-pH-Wert: 5,76 bei 30$^O$C

Unter den Bedingungen gemäß Beispiel 1 werden 190 µl CAM zum Starten der Reaktion eingesetzt; die Reaktion ist nach 40 Minuten und einem Verbrauch von 2,12 ml Na OH (0,5 m) beendet.
Die Aufarbeitung erfolgt wie in Beispiel 1 beschrieben. Die Partikelgröße beträgt ca. 2 - 5 µm.

B E I S P I E L    12

Anti-human Breitband-Partikel

In der in Beispiel 1 beschriebenen Versuchsanordnung werden folgende Komponenten gemischt:

8,4 ml Anti-human-Breitband vom Schaf
21,6 ml Na Phosphatpuffer (0,5 m, pH 5,5)

Ausgangs-pH-Wert: 5,72 bei 30$^O$C

Unter den Bedingungen gemäß Beispiel 1 werden 190 µl CAM zum Starten der Reaktion eingesetzt; die Reaktion ist nach 45 Minuten und einem Verbrauch von 2,20 ml Na OH (0,5 m) beendet.

Die Aufarbeitung erfolgt wie in Beispiel 1 beschrieben. Die Partikelgröße beträgt ca. 1 - 4 µm.

Die Partikel dienen zur Absorption verschiedener Plasmaproteine aus humanem Leichtkette-Kappa-Antigen.


# B E I S P I E L    13


### Human-Mischserum-Partikel


In der in Beispiel 1 beschriebenen Versuchsanordnung werden folgende Komponenten gemischt:

13,15 ml humanes Mischserum (ABO)

16,85 ml Na Phosphatpuffer (0,5 m, pH 5,5)

Ausgangs-pH-Wert: 5,95 bei 30°C

Unter den Bedingungen gemäß Beispiel 1 werden 200 µl CAM zum Starten der Reaktion eingesetzt; die Reaktion ist nach 25 Minuten und einem Verbrauch von 1,96 ml Na OH (0,5 m) beendet.

Die Aufarbeitung erfolgt wie in Beispiel 1 beschrieben. Die Partikelgröße beträgt ca. 50 µm.

(30 - 70) Die Partikel dienen zur Absorption mit Antigentestreagenz interferierender Komponenten des Humanserums. Das absorbierte Serum kann zur Herstellung der negativen Kontrollen verwendet werden.

Patentansprüche für AT, BE, CH, DE, FR, GB, IT, LI, LU, NL, SE

1.) Verfahren zur Herstellung von sphärischen Proteinpartikeln durch Polymerisation von Proteinen mittels Chlorameisensäurealkylestern in wässriger Lösung, dadurch gekennzeichnet, daß man den Proteingehalt der zu polymerisierenden wässrigen Proteinlösung im Bereich von 0,5 bis 4,5 Gew.%, den pH-Wert der Lösung im Bereich von 5,0 bis 6,0 und die Reaktionstemperatur im Bereich von 20-40°C hält und die Polymerisationsreaktion unter starkem Rühren durchführt.

2.) Verfahren nach Anspruch 1, dadurch gekennzeichnet, daß man das Reaktionsgefäß erdet.

3.) Verfahren gemäß Anspruch 1 und 2, dadurch gekennzeichnet, daß der pH-Wert etwa 5,5 und die Reaktionstemperatur etwa 30°C beträgt.

4.) Verfahren nach Anspruch 1 bis 3, dadurch gekennzeichnet, daß der Proteingehalt der zu polymerisierenden wässrigen Proteinlösung bei 2,5 Gew.% liegt.

5.) Sphärische Proteinpartikel mit einem Durchmesser von 1 bis 10 µm, hergestellt gemäß Verfahren nach den Ansprüchen 1 bis 3.